# EUROPEAN PATENT APPLICATION

(11) **EP 3 769 673 A1**
(43) Date of publication of application: **27.01.2021**
(21) Application number: 20187047.4
(22) Date of filing: 25.06.2014
(51) Int. Cl.: A61B 5/022, A61B 5/0235, G06Q 50/22

(54) **DEVICES AND METHODS FOR MEASURING BLOOD PRESSURE**

(30) Priority: 25.06.2013 US 201361838976 P; 24.06.2014 US 201414313713
(62) Divisional of application: 14818159.7
(71) Applicant: Qardio, Inc., San Francisco, CA 94104 (US)
(72) Inventor: FITZSIMONS, Duncan, London, W10 5LZ (GB); GAGGERO, Clara, London, E2 6DR (GB); WESTAWAY, Adrian, London, E2 6DR (GB); PELUSO, Marco, London, SW1X 0AX (GB); IANNELLA, Rosario, 1015 HB Amsterdam (NL)
(74) Representative: J A Kemp LLP

(57) **Abstract**

A portable blood pressure monitoring device capable of wirelessly interacting with a secondary device, such as a cell phone, to measure blood pressure of a patient wherein the device is configurable to fold into a low profile storage configuration. The blood pressure monitoring device includes a cuff for wrapping around the limb of a subject the cuff further having a an inflatable bladder formed integrally therein, wherein the cuff is substantially rectangular in shape having a first surface, a second surface, a first end, a second end, and two sides, and a securement mechanism; one or more tubes positioned within the cuff in communication with the inflatable bladder; an exhaust valve; a displayless housing connected to one of a first end and a second end of the cuff wherein the housing includes one or more of a controller, a memory, a pump, a power supply, and a motor; and a transmitter for wirelessly transmitting blood pressure data to a second device.

## Description

### CROSS-REFERENCE

This application claims priority to U.S. Utility Application No. 14/313,713 filed June 24, 2014, and U.S. Provisional Application No. 61/838,976, filed June 25, 2013, entitled DEVICES AND METHODS FOR MEASURING BLOOD PRESSURE by Duncan Fitzsimmons, et al., which applications are incorporated herein by reference.

### BACKGROUND OF THE INVENTION

Regular measurement of blood pressure is an important part of antihypertensive or hypotensive therapy, as well as the control of certain side-effects caused by certain therapies for various kinds of applications. According to the guidelines for antihypertensive therapies of the World Health Organization ("WHO"), the degrees of hypertension are classified in accordance with blood pressure values measured every 5 mmHg, and therapeutic methods suitable for the individual degrees are recommended. Therefore, whether appropriate therapies can be performed depends on the measured blood pressure values. Also, as the aging population advances, demands for high accuracy and high reliability of blood pressure measurement are on the rise in order to prevent circulatory organ diseases and metabolic syndrome which is impacted by hypertension.

Conventionally, a non-invasive sphygmomanometer for measuring the blood pressure uses a cuff wound around an arm of the patient, gradually changing the cuff pressure from a pressure higher than the systolic blood pressure (also called a maximum blood pressure) to a pressure lower than the diastolic blood pressure (also called a minimum blood pressure). A microphone method that measures the blood pressure by detecting the Korotkoff sounds as in the auscultatory method, achieved by listening, such as through a stethoscope, and the oscillometric method that measures the blood pressure by detecting the change in pulse wave superposed on the internal pressure of an internal air bladder of a cuff are used.

Currently, more than 50 million people in the United States suffer from high blood pressure, also referred to as hypertension. Internationally, about 1 in 5 people suffer from high blood pressure. Blood pressure has been called the silent killer because patients often do not detect symptoms while damage to the heart, brain and kidneys occurs. Controlling blood pressure can be achieved by a variety of medications, such as ACE inhibitors, alpha blockers, AR2 blockers, beta blockers, Calcium blockers, diuretics, and vasodilators. Additionally, a variety of life style changes can be employed to control blood pressure.

Patients diagnosed with high blood pressure are advised to engage in a regime of home monitoring - typically once or twice a day - in order to quickly identify changes in blood pressure control. A number of devices are available for home blood pressure measurement. Digital blood pressure devices are considered easy to use: they automatically calculate the pulse and display the systolic and diastolic pressures. However, a simple upper arm blood pressure cuff with a stethoscope is often considered by doctors the most reliable way to measure blood pressure. Omron Healthcare has a variety of products. Additionally Omron Healthcare holds several patents for blood pressure measurement devices including, for example, U.S.Patent US 4,776,344 A issued October 11, 1988, for *Electronic Blood Pressure Measuring Device;* US 7,794,405 B2 issued September 14, 2010, for *Cuff for Blood Pressure Monitor, and Blood Pressure Monitor Having the Same;* U.S. Pub US 2013/0138000 A1 published May 30, 2013, for *Blood Pressure Measurement Device.*

Traditionally, blood pressure monitors are based on an analog dial pressure measuring gauge, digital blood pressure monitors replace the analog dial with a liquid crystal display (LCD display) which is capable of showing mostly alphanumeric information. The traditional blood pressure monitor shows the changes in pressure up and down as the blood pressure cuff is first inflated and then deflated during the measurement. Typical blood pressure device are made of a central device unit tethered to an inflatable cuff by way of a flexible air pipe. This approach causes a disorderly storage of the unit when not in use, an unnecessarily large footprint, and is not practical for travel and general daily use. This issue is often mitigated with the introduction of a travel/storage pouch or case, to enclose the blood pressure device and all its parts. Certain blood pressure monitors are integrated with software on smartphone or other digital devices featuring advanced displays, capable of showing any kind of text or graphic information. Existing devices display continuously the pressure information throughout the measurement, causing the subject to become nervous and hence potentially affecting the equality of the measurement itself.

Technology has come a long way to enable patients to monitor their blood pressure in home. However, research suggests that up to one-third of people who were thought to have treatment-resistant may actually have "white coat hypertension"which results in blood pressure spiking in the doctor's office. Patients also see increases in blood pressure when stressed. One source of stress can be the anxiety associated with checking the blood pressure and concerns over the potential results.

What is needed is a convenient way for patients to test blood pressure remote from a clinic setting (e.g., at home or work) which also provides a way to reduce stress associated with the process. The device should be easy to store and use to facilitate convenient use in a plurality of locations.

### SUMMARY OF THE INVENTION

Devices and methods are described to enable a patient to conveniently test blood pressure remote from a clinic setting (e.g., a non-clinical setting such as at home or work) which provides a way to reduce stress associated with the process. The devices and methods are also adaptable to provide resulting information via a communication network to a remote location and/or a third party (health care provider, health buddy, etc.).

An aspect of the disclosure is directed to a blood pressure monitor device. The blood pressure monitoring device comprises: a cuff for wrapping around a limb of a subject the cuff further having a an inflatable bladder formed integrally therein, wherein the cuff is substantially rectangular in shape having a first surface, a second surface, a first end, a second end, and two sides, and a securement mechanism; one or more tubes positioned within the cuff in communication with the inflatable bladder; an exhaust valve; a displayless housing connected to one of a first end and a second end of the cuff wherein the housing includes one or more of a controller, a memory, a pump, a power supply, and a motor; and a transmitter for wirelessly transmitting blood pressure data to a second device. Additionally, one or more of an on-off button and a start-stop button can be provided on the device. Alternatively, the device can be activated by disengaging the cuff from the device body or by a software app on an electronic device in communication with the blood pressure monitor. Additionally, the blood pressure monitoring device can comprise a pulley. The pulley can be configured to engage the housing along an end and further wherein movement of the pulley away from the housing activates the monitoring device from an off condition to an on condition. In some configurations, the blood pressure monitoring device can comprise one or more of each of a pressure detector, a pulse detector and wireless sensors. A securement mechanism can be provided which secures the cuff in a position around the body of the blood pressure monitoring device when not in use. Suitable securement mechanisms include, for example, one or more magnets, and hook and loop fasteners. The blood pressure monitoring device is configurable to be in wireless communication with one or more display devices.

An additional aspect of the disclosure is directed to a method for measuring blood pressure The methods comprise: applying a blood pressure monitor having a cuff for wrapping around a limb of a subject the cuff having a an inflatable bladder formed integrally therein, wherein the cuff is substantially rectangular in shape having a first surface, a second surface, a first end, a second end, and two sides, and a securement mechanism; one or more tubes positioned within the cuff in communication with the inflatable bladder; an exhaust valve; a displayless housing connected to one of a first end and a second end of the cuff wherein the housing includes one or more of a controller, a memory, a pump, a power supply, and a motor; and a transmitter for wirelessly transmitting blood pressure data to a second device; and wirelessly delivering an output of the blood pressure monitor to a second device. Additional method steps include, for example, turning the blood pressure monitor on, either automatically, semi-automatically or manually. Automatic or semi-automatic activation can occur by, for example, extending the cuff away from the body of the blood pressure monitoring device, changing the device from a storage condition to a deployed condition, activating an application residing on an electronic device in communication with the blood pressure monitoring device. In some configurations, the blood pressure monitoring device transitions from an off condition to an on condition by moving a pulley on the blood pressure monitoring device housing from a first position to a second position (e.g., rotating the pulley away from the device housing). Additionally, the method includes measuring one or more of a blood pressure of a patient or user, and a pulse of a patient or user. An acquired blood pressure measurement and/or pulse measurement can be provided to a third party device that is different than the second device (e.g. a device associated with a healthcare provider). The step of providing an acquired blood pressure measurement and/or pulse measurement to a third party can occur automatically, semi-automatically or manually. Additionally, the step of providing an acquired blood pressure measurement and/or pulse measurement to a third party can occur wirelessly.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**FIGS. 1A-H** are views of a blood pressure cuff which engages a device body housing the blood pressure testing electronics;
**FIGS. 2A-B** are perspective views of a blood pressure cuff which is capable of wrapping around the device body to provide a low-profile and portable storage configuration;
**FIGS. 3A-B** are side views illustrating a pulley element which attaches to the device body;
**FIGS. 4A-B** are side views illustrating the cuff in place around the device body;
**FIGS. 5A-B** are side views of an electronic pump and wireless communication system inside the main device body;
**FIG. 6** illustrates a textile cuff assembled from layers of textile and internal components; and
**FIGS. 7A-B** illustrate a communication network through which the devices disclosed can communicate data.

### DETAILED DESCRIPTION OF THE INVENTION

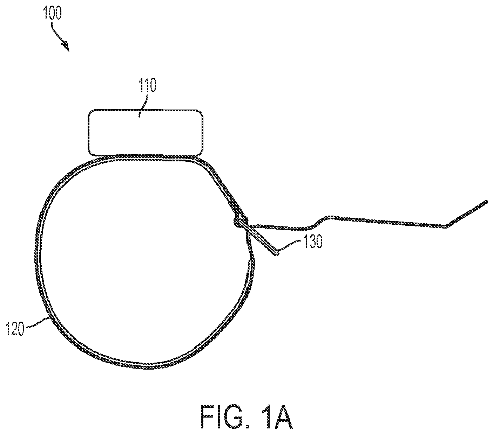

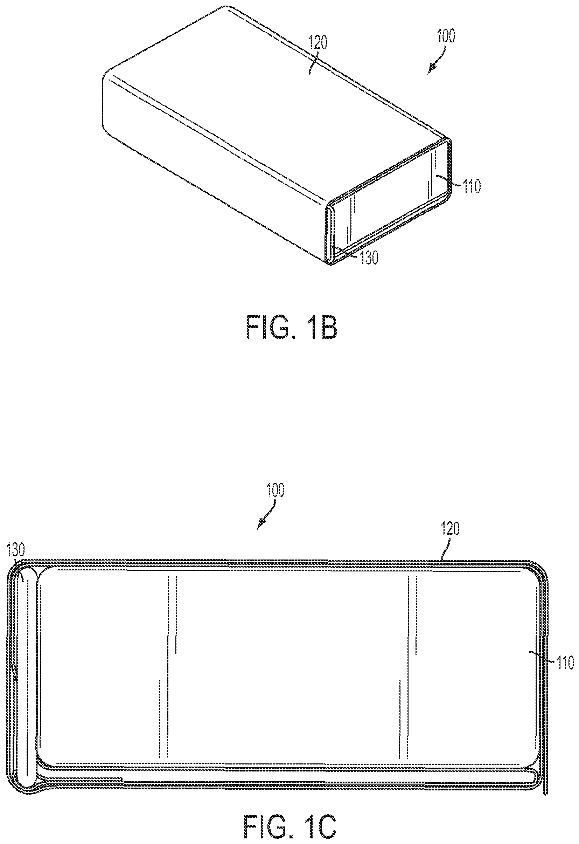

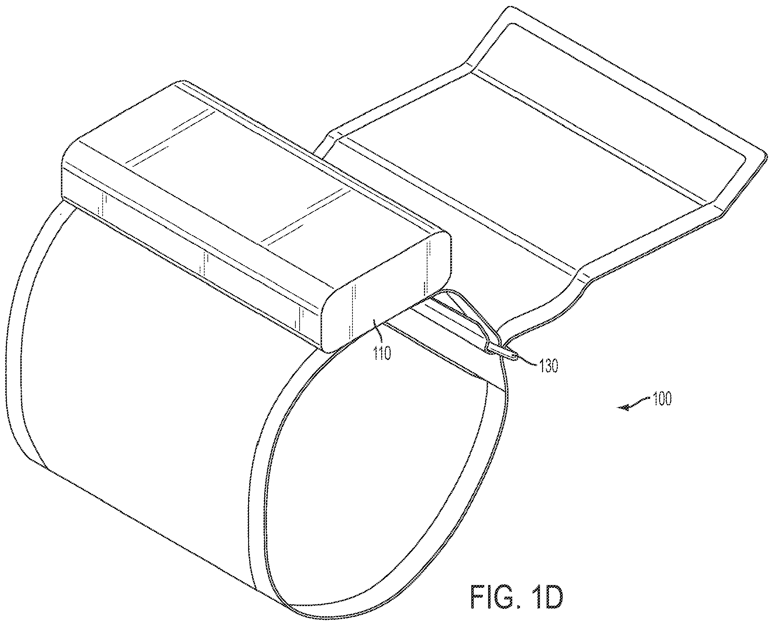

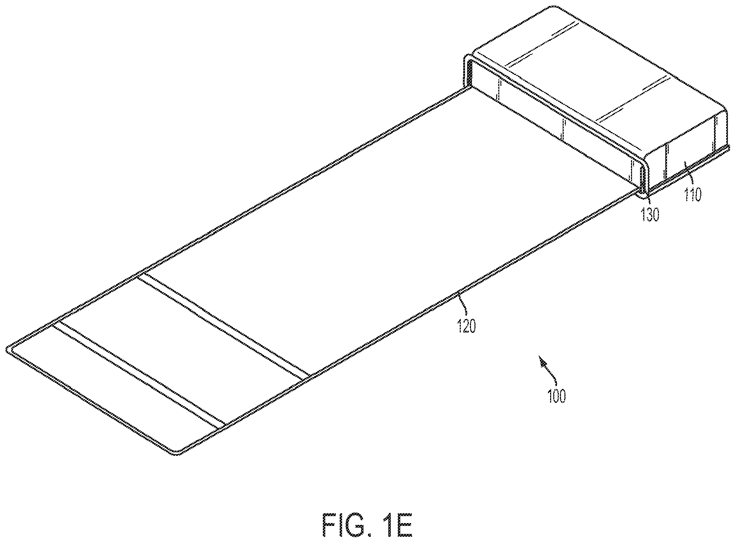

**FIGS. 1A-H** are views of a blood pressure measurement device ***100*** which has a device body ***110*** which is configured to house the electronics required to measure blood pressure. The blood pressure measurement device ***100*** includes a cuff member ***120*** comprising, for example, a compression air bladder, a sub air bladder, and a pulse wave detection air bladder, pressure controller for pressurizing or depressurizing each air bladder, a pressure sensor which is capable of sensing an internal pressure of each air bladder, pulse wave signal extractor for extracting time-series data of a pulse wave signal superposed on a cuff internal pressure. A buckle or pulley ***130*** is provided through which the cuff member ***120*** can pass for securement around the arm of a user. When the cuff ***120*** is unrolled from around the device body ***110,*** the device can turn on; similarly when the cuff ***120*** is secured around the device body ***110*** (e.g., for storage), the device can automatically turn off.

When the pressure controller in the device body ***110*** pressurizes or depressurizes each air bladder of the cuff member ***120*,** a blood pressure value deriver capable of deriving a systolic blood pressure value and/or a diastolic blood pressure value based on a change in a feature amount of the pulse wave signal and a cuff internal pressure at a point of time of the change. Any wires, tubes or interface elements between the device body ***110*** and the cuff member ***110*** are not visible to the user.

To pressurize and depressurize the cuff member ***120*** which is adapted to be secured around (pulled tight), for example, a patient's arm, a pump is provided which has a connector which enables pressurizing/depressurizing connected to a compression air bladder of the cuff main body via a second tube and tube, to a pulse wave detection air bladder of the cuff main body via a first tube and fluid resistor, and to the sub air bladder of the cuff main body via a third tube and opening/closing valve. Also, a pressure sensor as a cuff pressure detector for obtaining a cuff pressure signal from the change in pressure of the pulse wave detection air bladder is connected to the pulse wave detection air bladder via the first tube. Furthermore, the third tube is connected to the sub air bladder.

The first, second, and third tubes, which are not visible to the user, are made of soft tubes. The third tube may be further connected to a damper device that increases the volume in proportion to the pressure and smooth the pressure. Other processes of pressurizing and depressurizing the cuff can be employed without departing from the scope of the disclosure.

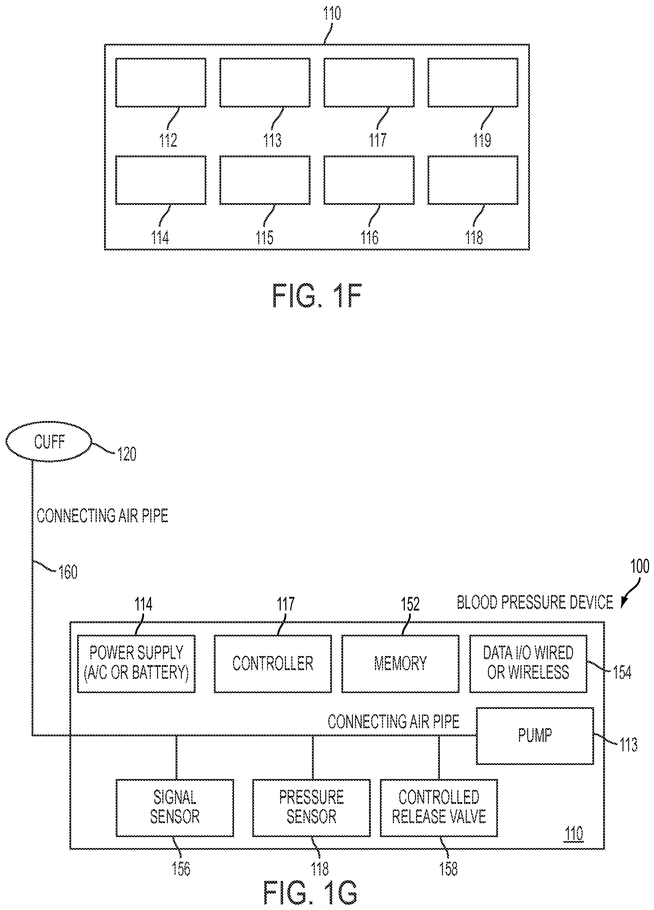

As shown in **FIG. 1F****,** the device body ***110*** is configurable to house the electronics which, for example, control the application of pressure to the cuff and analyze the resulting pressure data as well as transmitting the data ***116*** (e.g. via Bluetooth) to another device. For example, the pump ***112*** may be driven by a suitable power supply ***114*** (e.g., battery) from a pump driver ***113*** connected to a motor ***115,*** and supplies the external air into the pump through an opening, thereby performing pressurization. A controller ***117*** is provided which controls the operation of the device. The air bladders of the cuff can then be pressurized by supplying the pressurizing air to a tube or bladder. A rapid exhaust valve/constant-rate exhaust valve can be provided that has a structure in which the opening area can be changed by the magnitude of the electromagnetic force in order to achieve a depressurization rate of 2 to 4 mmHg/sec.

A pressure sensor ***118*,** such as a cuff pressure detector, is configurable to receive a compression pressure signal from the compression air bladder, in which the pulse wave component is attenuated via the fluid resistor, and the pressure change of the pulse wave detection air bladder. A pressure measuring unit ***119*** for conversion into an analog electrical signal may be provided which is connected to the pressure sensor, and an A/D converter is connected to the pressure measuring unit. The A/D converter can then output a digital signal as a cuff pressure signal to a central controller ***117*.**

The central controller ***117*** is configurable to include a random access memory (RAM) for performing, for example, read and write of measurement data and analytical results, and a read only memory (ROM) for storing, as various control programs readable by the central controller, a pulse wave processor for detecting a pulse wave signal superposed on a cuff pressure signal, a cuff pressure controller for pressurizing and depressurizing the cuff (the compression air bladder, pulse wave detection air bladder, and sub air bladder), a blood pressure measuring unit for determining the blood pressure from the detected pulse wave change and compression cuff pressure signal. As would be evident to a person of skill in the art, the RAM can also function as a work area of programs to be processed by the central controller ***117*.**

The central controller ***117*** is also in communication with a secondary electronic device ***150*** such as a mobile device which can act as a blood pressure display for displaying the final blood pressure value, and to drivers for performing the above-mentioned driving control operations. The power supply from a power supply unit, including batteries, is performed such that the central controller can perform each operation required for blood pressure measurement by supplying power to the corresponding unit in accordance with the operation of a switch.

As shown in **FIG. 1G****,** a blood pressure monitoring device ***100*** has a device body ***110*** which houses a power supply ***152*,** a controller ***117*,** a memory ***152,*** a data i/o ***154*** which is wired or wireless, a signal sensor ***156*,** a pressure sensor ***118***, a controlled release valve ***158*,** and a pump ***113*** which is connected to the cuff ***120*** by a connecting air pipe ***160**.*

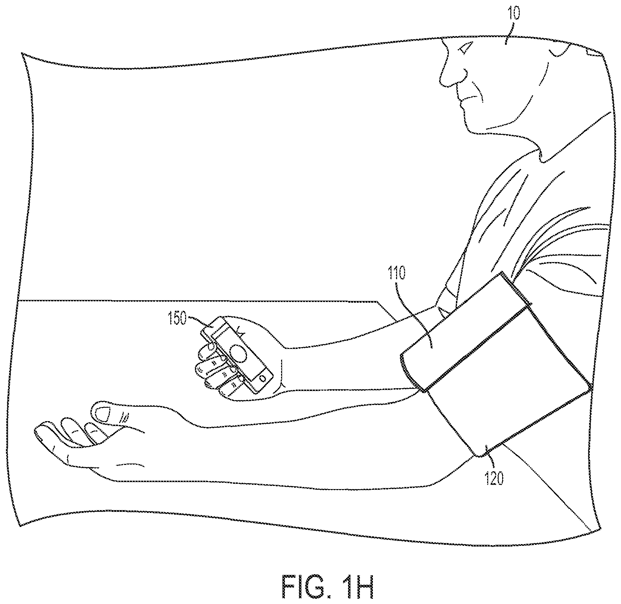

As shown in **FIG. 1H****,** a user ***10*** has a blood pressure monitoring device ***100*** comprising a blood pressure cuff ***120*** and a device body ***110*** secured about the arm of the user ***10**.* The blood pressure monitoring device *100* is in wireless communication with a secondary electronic device ***150**,* such as a smart phone.

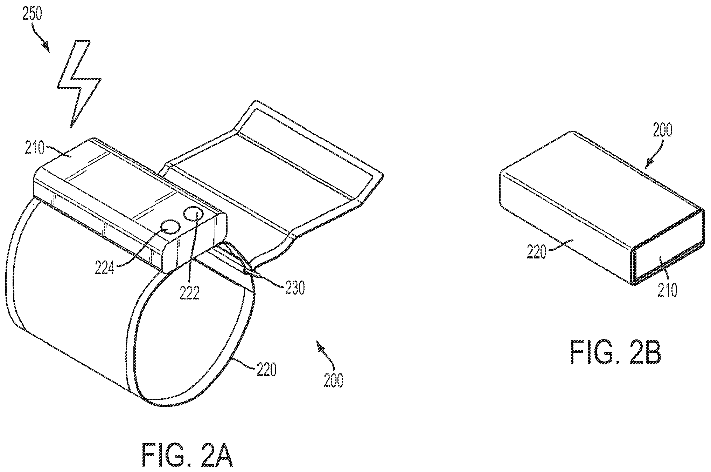

**FIGS. 2A-B** are a perspective view of a blood pressure monitoring device 200, in an open and closed configuration, which includes a blood pressure cuff ***220*** which is capable of wrapping around the device body ***210*** to provide a low-profile and portable storage configuration. The blood pressure cuff ***220*** is configurable to have an on/off button ***222*** and/or a start/stop button ***224*** for taking a measurement. As will be appreciated the on/off button can include the functionality of start/stop. However, unlike other devices, a display and wires are not provided on the device body ***210*.** Rather, the measurements can be taken wirelessly and transmitted to a secondary device ***250*,** such as a mobile phone, which can display the final results. As will be appreciated by those skilled in the art, the device body ***210*** is illustrated in a rectangular form factor. However, other shapes can be employed without departing from the scope of the invention. For example, the device body can be any suitable shape in two dimensions, such as square, round, oval, ovoid, etc. The device body **210** can also be any suitable shape in three dimension, such as cube, etc. Other shape variations will be apparent to those skilled in the art.

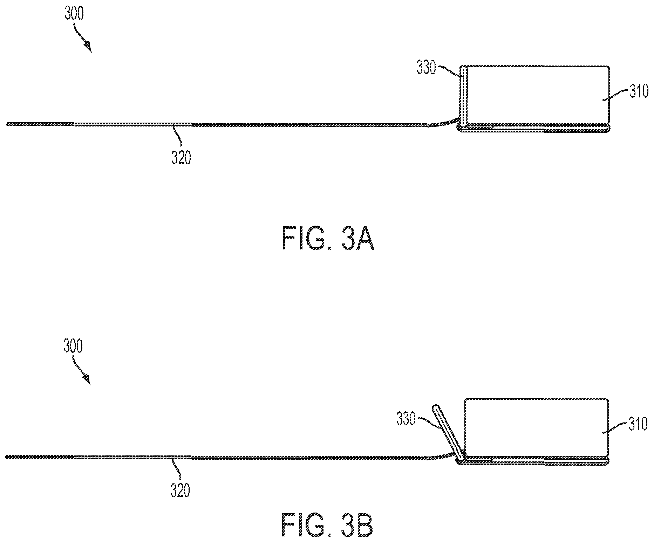

**FIG. 3** is a side view illustrating a blood pressure monitoring device ***300*** where a pulley element ***330*** attaches to the device body ***310.*** The pulley element ***330*** is illustrated attached at an end of the device body ***310.*** Attachment can be achieved by any suitable mechanism, such as magnets or hinges, etc. The pulley ***330*** is rotated away from the device body ***310*** to make the cuff ***320*** available for use. The pulley ***330*** can also be used to activate the electronic elements of the device, in lieu of an on/off button or start/stop button. Thus, for example, the process of rotating the pulley ***330*** away from the device body ***310*** causes activation of the device. Additionally, in at least some configurations, activating the pulley ***330*** can be used to activate electronic elements of the device and/or wireless sensors (such as a reed switch, magnetic switch or light dependent resistor ("LDR")). As will be appreciated by those skilled in the art, these electronic elements that activate the device can be magnetic switches (detecting movement of magnets embedded in the cuff), light dependent resistors (detecting light deriving from the opening of the cuff), and/or a near field communication ("NFC") element (detecting proximity of another NFC element embedded in the cuff), or it is also imaginable a low-power design that is always on and requires no such elements at all.

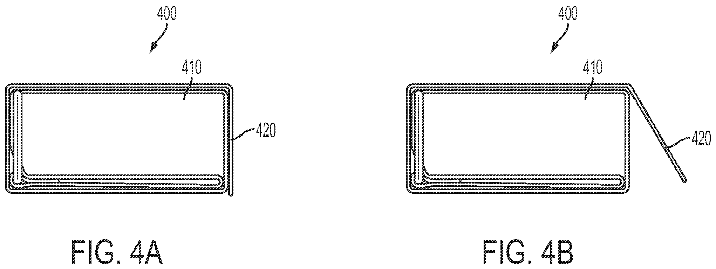

**FIG. 4** is a side view illustrating a blood pressure monitoring device ***400*,** where the cuff ***420*** in place around the device body ***410*.** As evident from this view, in a storage configuration with the pulley ***430*** engaged, the device ***400*** takes on a low profile without tubes and wires extending from the device making the device ***400*** easy to store and transport. The distal end of the cuff can be secured by the use of a securement mechanism such as magnets or a hook and loop fastener such as Velcro® brand fasteners (available from Velcro Industries B.V. or similar fastening technologies available by other manufacturers) As will be appreciated by those skilled in the art, the cuff length vary depending on the characteristic of the subject being measured. Typically for adults, for example, the width of the cuff would be between 13 and 15cm, and the length of the cuff would be sized to accommodate an upper-arm circumference between 22cm and 32cm - and thus would be longer than 22cm - 32cm to provide additional length for wrap-around and securement.

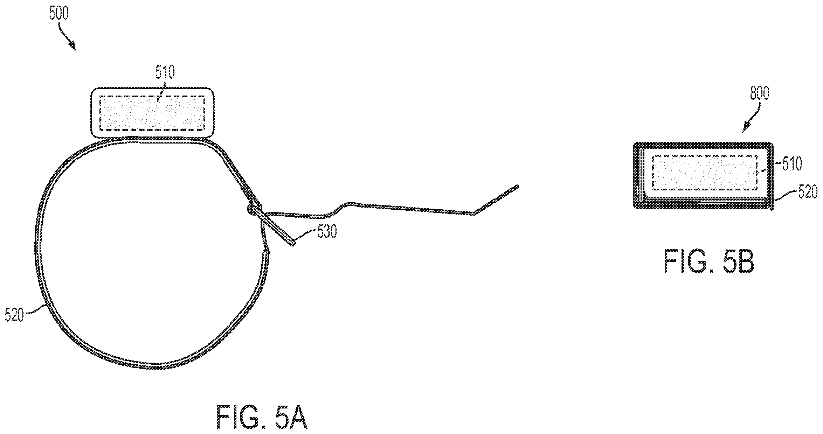

**FIG. 5** is a side view illustrating a blood pressure monitoring device ***500**,* with an electronic pump and pressure sensors and wireless communication system inside the main device body ***510**.* The electronics and mechanical components, discussed above, are contained within the housing ***510**.* The method is suitable for application to blood pressure measurements to the upper arm or the wrist. All electronic and mechanical components are secured within the device body which are seamlessly connected to the cuff ***520*** component. In this configuration the pulley ***530*** is positioned away from the device body ***510**.*

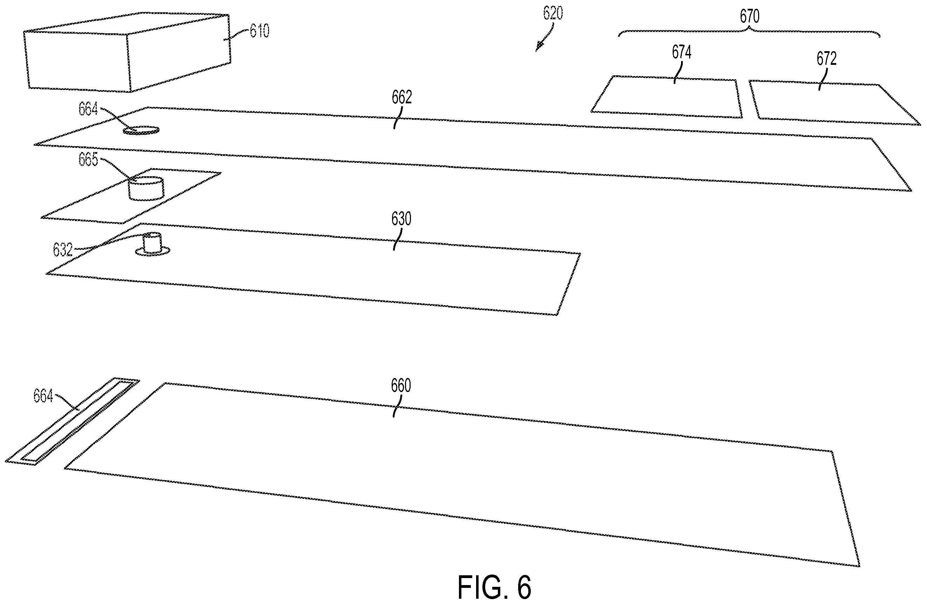

**FIG. 6** illustrates an example of a textile cuff ***620*** assembled from layers of textile and internal components. As illustrated, the cuff ***620*** is elongate layer ***621*** which has a first flexible layer ***660*** and a second flexible layer ***662***. Flexible layers can, for example, be fabric layers. A securement mechanism ***670*** such as a hook and loop fastener (e.g., Velcro®) comprising a first hook fastener section ***672*** and a second loop fastener section ***674***. Magnetic elements can be also provided which assist in securing the cuff in a closed position when not in use and achieving a low profile during storage and/or facilitate identification of an "on" or "off' state for the device.. Semi-rigid mechanical fixing elements can also be provided. As will be understood by persons of skill in the art, blood pressure monitors include: a pump (that inflates the cuff located inside the device ***610*** and illustrated in **FIG. 1G**), a controlled release valve (that lets the air out at a controlled rate), a pressure gauge and a signal sensor (a microphone, really). As shown in **FIG. 6**, an aperture ***664*** is provided in the flexible layer ***662*** which enables a mechanical connector ***665*** to pass through the layer and connect to the device. The mechanical connector facilitates a secure connection of the cuff to the monitoring device. An inflatable bladder ***630*** which has an air connector ***632*** that connects the bladder ***530*** to the air pipes of the monitoring device. The flexible layer ***660*** can further include, for example a metal ring ***661*** which allows the cuff to be looped around the upper arm of a patient during use. During deflation, when the signal sensor starts hearing the heartbeat, it means that blood pressure ("BP") is less than or equal to the current air pressure in the cuff (systolic BP). When the signal sensors stop hearing the heartbeat, it means that blood pressure is greater than or equal to the current air pressure in the cuff (diastolic BP). This effectively means that the blood pressure is only sampled at each heartbeat. Slightly different algorithms can be applied as well, for example, sampling the BP during inflation (instead of during deflation). In this second case, when, during inflation the signal sensor starts hearing the heartbeat, it means that blood pressure is greater than or equal to the current air pressure in the cuff (diastolic BP). When the signal sensors stop hearing the heartbeat, it means that blood pressure is lower than or equal to the current air pressure in the cuff (systolic BP). This second method is faster, as it takes less time to inflate and deflate the cuff, but it is potentially less reliable due to the noise caused by the pump potentially interfering with the heart beat detection.

The systems and methods described herein rely on a variety of computer systems, networks, digital devices, and/or software apps for operation. In order to fully appreciate how the system operates an understanding of suitable computing systems is useful. The systems and methods disclosed herein are enabled as a result of application via a suitable computing system.

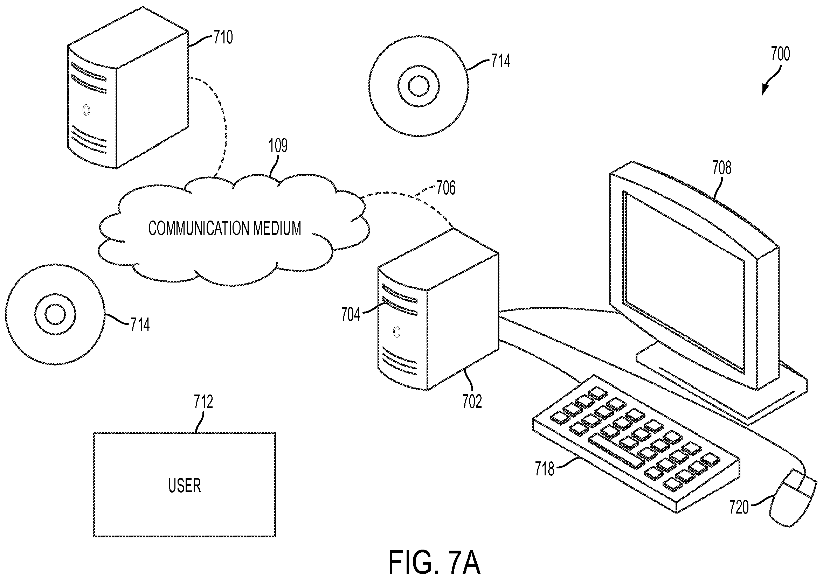

**FIG. 7A** is a block diagram showing a representative example logic device through which the display from the monitor can be achieved. A computer system (or digital device) ***700**,* which may be understood as a logic apparatus adapted and configured to read instructions from media ***714*** and/or network port ***706**,* is connectable to a server ***710**,* and has a fixed media ***716***. The computer system ***700*** can also be connected to the Internet or an intranet. The system includes central processing unit (CPU) ***702**,* disk drives ***704**,* optional input devices, illustrated as keyboard ***718*** and/or mouse ***720*** and optional monitor ***708**.* Data communication can be achieved through, for example, communication medium ***709*** to a server ***710*** at a local or a remote location. The communication medium ***709*** can include any suitable means of transmitting and/or receiving data. For example, the communication medium can be a network connection, a wireless connection or an internet connection. It is envisioned that data relating to the present invention can be transmitted over such networks or connections. The computer system can be adapted to communicate with a participant and/or a device used by a participant. The computer system is adaptable to communicate with other computers over the Internet, or with computers via a server.

The computing system ***700*** can be configured such that it is capable of executing a variety of computing applications ***738***, including computing applications, a computing applet, a computing program, or other instructions for operating on computing system ***703*** to perform at least one function, operation, and/or procedure. Computing system ***703*** is controllable by computer readable storage media for tangibly storing computer readable instructions, which may be in the form of software. The computer readable storage media adapted to tangibly store computer readable instructions can contain instructions for computing system ***703*** for storing and accessing the computer readable storage media to read the instructions stored thereon themselves. Such software may be executed within CPU to cause the computing system to perform desired functions. In many known computer servers, workstations and personal computers CPU is implemented by micro-electronic chips CPUs called microprocessors. Optionally, a co-processor, distinct from the main CPU, can be provided that performs additional functions or assists the CPU. The CPU may be connected to co-processor through an interconnect. One common type of coprocessor is the floating-point coprocessor, also called a numeric or math coprocessor, which is designed to perform numeric calculations faster and better than the general-purpose CPU.

As will be appreciated by those skilled in the art, a computer readable medium stores computer data, which data can include computer program code that is executable by a computer, in machine readable form. By way of example, and not limitation, a computer readable medium may comprise computer readable storage media, for tangible or fixed storage of data, or communication media for transient interpretation of code-containing signals. Computer readable storage media, as used herein, refers to physical or tangible storage (as opposed to signals) and includes without limitation volatile and non-volatile, removable and non-removable storage media implemented in any method or technology for the tangible storage of information such as computer-readable instructions, data structures, program modules or other data. Computer readable storage media includes, but is not limited to, RAM, ROM, EPROM, EEPROM, flash memory or other solid state memory technology, CD-ROM, DVD, or other optical storage, magnetic cassettes, magnetic tape, magnetic disk storage or other magnetic storage devices, or any other physical or material medium which can be used to tangibly store the desired information or data or instructions and which can be accessed by a computer or processor.

Some embodiments may be implemented in one or a combination of hardware, firmware and software. Embodiments may also be implemented as instructions stored on a non-transitory computer-readable storage medium, which may be read and executed by at least one processor to perform the operations described herein. A non-transitory computer-readable storage medium may include any mechanism for storing information in a form readable by a machine (e.g., a computer). For example, a non-transitory computer-readable storage medium may include read-only memory (ROM), random-access memory (RAM), magnetic disk storage media, optical storage media, flash-memory devices, and other non-transitory media.

In operation, the CPU fetches, decodes, and executes instructions, and transfers information to and from other resources via the computer's main data-transfer path, system bus. Such a system bus connects the components in the computing system and defines the medium for data exchange. Memory devices coupled to the system bus include random access memory (RAM) and read only memory (ROM). Such memories include circuitry that allows information to be stored and retrieved. The ROMs generally contain stored data that cannot be modified. Data stored in the RAM can be read or changed by CPU or other hardware devices. Access to the RAM and/or ROM may be controlled by memory controller. The memory controller may provide an address translation function that translates virtual addresses into physical addresses as instructions are executed.

In addition, the computing system can contain peripherals controller responsible for communicating instructions from the CPU to peripherals, such as, printer, keyboard, mouse, and data storage drive. Display, which is controlled by a display controller, is used to display visual output generated by the computing system. Such visual output may include text, graphics, animated graphics, and video. The display controller includes electronic components required to generate a video signal that is sent to display. Further, the computing system can contain network adaptor which may be used to connect the computing system to an external communications network.

As is well understood by those skilled in the art, the Internet is a worldwide network of computer networks. Today, the Internet is a public and self-sustaining network that is available to many millions of users. The Internet uses a set of communication protocols called TCP/IP (i.e., Transmission Control Protocol/Internet Protocol) to connect hosts. The Internet has a communications infrastructure known as the Internet backbone. Access to the Internet backbone is largely controlled by Internet Service Providers (ISPs) that resell access to corporations and individuals.

The Internet Protocol (IP) enables data to be sent from one device (e.g., a phone, a Personal Digital Assistant (PDA), a computer, etc.) to another device on a network. There are a variety of versions of IP today, including, e.g., IPv4, IPv6, etc. Other IPs are no doubt available and will continue to become available in the future, any of which can be used without departing from the scope of the invention. Each host device on the network has at least one IP address that is its own unique identifier and acts as a connectionless protocol. The connection between end points during a communication is not continuous. When a user sends or receives data or messages, the data or messages are divided into components known as packets. Every packet is treated as an independent unit of data and routed to its final destination - but not necessarily via the same path.

The Open System Interconnection (OSI) model was established to standardize transmission between points over the Internet or other networks. The OSI model separates the communications processes between two points in a network into seven stacked layers, with each layer adding its own set of functions. Each device handles a message so that there is a downward flow through each layer at a sending end point and an upward flow through the layers at a receiving end point. The programming and/or hardware that provides the seven layers of function is typically a combination of device operating systems, application software, TCP/IP and/or other transport and network protocols, and other software and hardware.

Wireless networks can incorporate a variety of types of mobile devices, such as, e.g., cellular and wireless telephones, PCs (personal computers), laptop computers, wearable computers, cordless phones, pagers, headsets, printers, PDAs, etc. For example, mobile devices may include digital systems to secure fast wireless transmissions of voice and/or data. Typical mobile devices include some or all of the following components: a transceiver (for example a transmitter and a receiver, including a single chip transceiver with an integrated transmitter, receiver and, if desired, other functions); an antenna; a processor; display; one or more audio transducers (for example, a speaker or a microphone as in devices for audio communications); electromagnetic data storage (such as ROM, RAM, digital data storage, etc., such as in devices where data processing is provided); memory; flash memory; and/or a full chip set or integrated circuit; interfaces (such as universal serial bus (USB), coder-decoder (CODEC), universal asynchronous receiver-transmitter (UART), phase-change memory (PCM), etc.). Other components can be provided without departing from the scope of the invention.

Wireless LANs (WLANs) in which a mobile user can connect to a local area network (LAN) through a wireless connection may be employed for wireless communications. Wireless communications can include communications that propagate via electromagnetic waves, such as light, infrared, radio, and microwave. There are a variety of WLAN standards that currently exist, such as Bluetooth®, IEEE 802.11, and the obsolete HomeRF.

By way of example, Bluetooth products may be used to provide links between mobile computers, mobile phones, portable handheld devices, personal digital assistants (PDAs), and other mobile devices and connectivity to the Internet. Bluetooth is a computing and telecommunications industry specification that details how mobile devices can easily interconnect with each other and with non-mobile devices using a short-range wireless connection. Bluetooth creates a digital wireless protocol to address end-user problems arising from the proliferation of various mobile devices that need to keep data synchronized and consistent from one device to another, thereby allowing equipment from different vendors to work seamlessly together.

An IEEE standard, IEEE 802.11, specifies technologies for wireless LANs and devices. Using 802.11, wireless networking may be accomplished with each single base station supporting several devices. In some examples, devices may come pre-equipped with wireless hardware or a user may install a separate piece of hardware, such as a card, that may include an antenna. By way of example, devices used in 802.11 typically include three notable elements, whether or not the device is an access point (AP), a mobile station (STA), a bridge, a personal computing memory card International Association (PCMCIA) card (or PC card) or another device: a radio transceiver; an antenna; and a MAC (Media Access Control) layer that controls packet flow between points in a network.

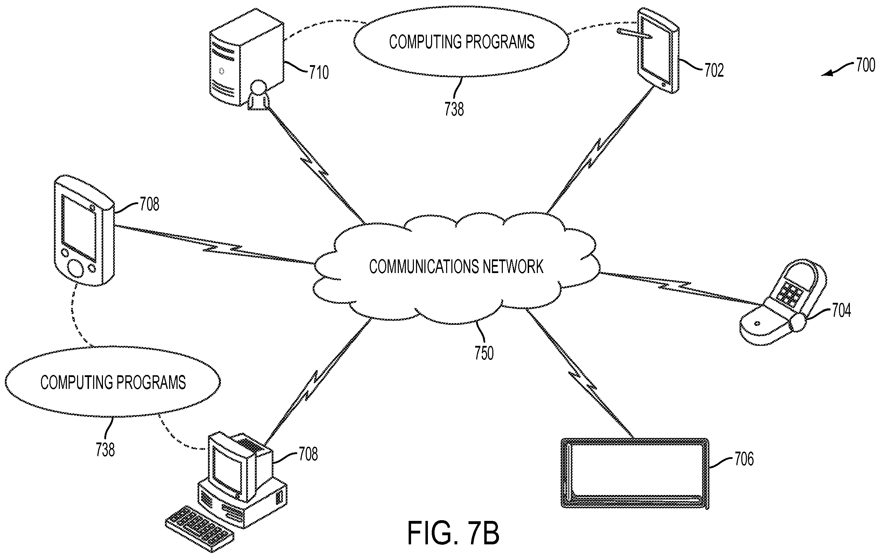

Computing system, described above, can be deployed as part of a computer network used to achieve the desired technical effect and transformation. In general, the above description for computing environments applies to both server computers and client computers deployed in a network environment. **FIG. 7B** illustrates an exemplary illustrative networked computing environment ***703,*** with a server in communication with client computers via a communications network ***750.*** As shown in **FIG. 7B****,** server ***710*** may be interconnected via a communications network ***750*** (which may be either of, or a combination of a fixed-wire or wireless LAN, WAN, intranet, extranet, peer-to-peer network, virtual private network, the Internet, or other communications network) with a number of client computing environments such as tablet personal computer, mobile telephone, smart phone, personal computer, and personal digital assistant. In a network environment in which the communications network ***750*** is the Internet, for example, server ***710*** can be dedicated computing environment servers operable to process and communicate data to and from client computing environments via any of a number of known protocols, such as, hypertext transfer protocol (HTTP), file transfer protocol (FTP), simple object access protocol (SOAP), or wireless application protocol (WAP). Other wireless protocols can be used without departing from the scope of the disclosure, including, for example Wireless Markup Language (WML), DoCoMo i-mode (used, for example, in Japan) and XHTML Basic. Additionally, networked computing environment ***703*** can utilize various data security protocols such as secured socket layer (SSL) or pretty good privacy (PGP). Each client computing environment can be equipped with operating system ***738*** operable to support one or more computing applications, such as a web browser (not shown), or other graphical user interface (not shown), or a mobile desktop environment (not shown) to gain access to server computing environment ***703.***

In operation, a user (not shown) may interact with a computing application running on a client computing environment to obtain desired data and/or computing applications. The data and/or computing applications may be stored on server computing environment ***703*** and communicated to cooperating users through client computing environments over exemplary communications network ***750.*** The computing applications, described in more detail below, are used to achieve the desired technical effect and transformation set forth. A participating user may request access to specific data and applications housed in whole or in part on server computing environment ***703.*** These data may be communicated between client computing environments and server computing environments for processing and storage. Server computing environment ***703*** may host computing applications, processes and applets for the generation, authentication, encryption, and communication data and applications and may cooperate with other server computing environments (not shown), third party service providers (not shown), network attached storage (NAS) and storage area networks (SAN) to realize application/data transactions.

### EXAMPLES

A user opens the blood pressure monitor from its storage configuration and secures the monitor to his or her arm such that the sensor is positioned to optimally detect blood pressure. The monitor is activated to begin detecting the user's blood pressure. Concurrently, or substantially concurrently, a display device (computer, mobile device, etc.) presents sensory input (visual, audio, or both) to the user during the process of obtaining the measurement. At the conclusion of obtaining the measurements, the results may be either one or both of presented to the user (by visual, audio or both) and presented to a third party (doctor, health buddy, etc.).

A user opens the blood pressure monitor from its storage configuration. Activating the pulley powers the device into a ready configuration. Thereafter the user secures the monitor to his or her arm such that the sensor is positioned to optimally detect blood pressure. The monitor is activated by a command provided by a device or computing system connected wirelessly to the blood pressure monitor. Such command may be user triggered (e.g. by pressing a button on a touch screen, or activating any other user interface element) or generated automatically by the system to begin detecting the user's blood pressure. Concurrently, or substantially concurrently, a display device (computer, mobile device, etc.) presents sensory input (visual, audio, or both) to the user during the process of obtaining the measurement. At the conclusion of obtaining the measurements, the results may be either one or both of presented to the user (by visual, audio or both) and presented to a third party (doctor, health buddy, etc.).

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.
The claims of the parent application are reproduced immediately below as clauses. These clauses define preferred embodiments. The applicant reserves the right to pursue protection for the combinations of features set out in these clauses, and/or for any other subject-matter contained in the parent application as filed, either in the present divisional application or in a further application divided from the present divisional application.
Clause 1. A blood pressure monitor device comprising:
   a cuff for wrapping around a limb of a subject the cuff further having a an inflatable bladder formed integrally therein, wherein the cuff is substantially rectangular in shape having a first surface, a second surface, a first end, a second end, and two sides, and a securement mechanism;
   one or more tubes positioned within the cuff in communication with the inflatable bladder;
   an exhaust valve;
   a displayless housing connected to one of a first end and a second end of the cuff wherein the housing includes one or more of a controller, a memory, a pump, a power supply, and a motor; and
   a transmitter for wirelessly transmitting blood pressure data to a second device.
Clause 2. The blood pressure monitoring device of clause 1 further comprising one or more of an on-off button and a start-stop button.
Clause 3. The blood pressure monitoring device of clause **1** further comprising a pulley.
Clause 4. The blood pressure monitoring device of clause **3** wherein the pulley engages the housing along an end and further wherein movement of the pulley away from the housing activates the monitoring device from an off condition to an on condition.
Clause 5. The blood pressure monitoring device of clause **1** further comprising a pressure detector.
Clause 6. The blood pressure monitoring device of clause **1** further comprising a pulse detector.
Clause 7. The blood pressure monitoring device of clause **1** further comprising one or more wireless sensors.
Clause 8. The blood pressure monitoring device of clause **1** wherein the securement mechanism is at least one of magnets and a hook and loop fastener.
Clause 9. The blood pressure monitoring device of clause **1** wherein the blood pressure monitoring device is in wireless communication with one or more displays.
Clause 10. The blood pressure monitoring device of clause **2** wherein the blood pressure monitoring device is in wireless communication with one or more displays.
Clause 11. A method for measuring blood pressure comprising:
   applying a blood pressure monitor having a cuff for wrapping around a limb of a subject the cuff having a an inflatable bladder formed integrally therein,
   wherein the cuff is substantially rectangular in shape having a first surface, a second surface, a first end, a second end, and two sides, and a securement mechanism; one or more tubes positioned within the cuff in communication with the inflatable bladder; an exhaust valve; a displayless housing connected to one of a first end and a second end of the cuff wherein the housing includes one or more of a controller, a memory, a pump, a power supply, and a motor;
   and a transmitter for wirelessly transmitting blood pressure data to a second device; and
   wirelessly delivering an output of the blood pressure monitor to a second device.
Clause 12. The method for measuring blood pressure of clause **11** further comprising the step of turning the blood pressure monitor on.
Clause 13. The method for measuring blood pressure of clause **12** wherein the step of turning the blood pressure monitor on is achieved by moving a pulley.
Clause 14. The method for measuring blood pressure of clause **11** further comprising measuring a blood pressure.
Clause 15. The method for measuring blood pressure of clause **11** further comprising measuring a pulse.
Clause 16. The method for measuring blood pressure of clause **11** comprising providing an acquired blood pressure measurement to a third party device that is different than the second device.
Clause 17. The method for measuring blood pressure of clause **16** wherein the step of providing an acquired blood pressure measurement to a third party occurs automatically.
Clause 18. The method for measuring blood pressure of clause **16** wherein the step of providing the acquired blood pressure measurement to a third party device occurs wirelessly.

## Claims

1. A blood pressure monitor device (100) comprising:
a cuff (120) for wrapping around a limb of a subject the cuff further having an inflatable bladder formed integrally therein,
wherein the cuff is substantially rectangular in shape having a first surface, a second surface, a first end, a second end, and two sides, and a securement mechanism;
one or more tubes positioned within the cuff in communication with the inflatable bladder;
an exhaust valve;
a housing (110) having an oval shape in one dimension and an on-off button, wherein the housing is connected to one of a first end and a second end of the cuff;
wherein the housing includes one or more of a controller, a memory, a pump, a power supply, and a motor; and
a transmitter for wirelessly transmitting blood pressure data to a second device.

2. The blood pressure monitoring device of claim 1 further comprising a pressure detector (118).

3. The blood pressure monitoring device of claim **1 or 2** further comprising a pulse detector.

4. The blood pressure monitoring device of any one of claims **1 to 3** further comprising one or more wireless sensors.

5. The blood pressure monitoring device of any one of claims **1 to 4** wherein the securement mechanism is at least one of magnets and a hook and loop fastener.

6. The blood pressure monitoring device of any one of claims **1 to 5** wherein the blood pressure monitoring device is in wireless communication with one or more displays.

7. A method for measuring blood pressure comprising:
applying a blood pressure monitor (100) having a cuff (120) for wrapping around a limb of a subject, the cuff having an inflatable bladder formed integrally therein, wherein the cuff is substantially rectangular in shape having a first surface, a second surface, a first end, a second end, and two sides, and a securement mechanism; one or more tubes positioned within the cuff in communication with the inflatable bladder; an exhaust valve; a housing (110) having an oval shape in one dimension and an on-off button, the housing connected to one of a first end and a second end of the cuff, wherein the housing includes one or more of a controller, a memory, a pump, a power supply, and a motor; and a transmitter for wirelessly transmitting blood pressure data to a second device;
turning the blood pressure monitor on via the on-off button; and
wirelessly delivering an output of the blood pressure monitor to a second device.

8. The method for measuring blood pressure of claim **7** further comprising measuring a blood pressure.

9. The method for measuring blood pressure of claim **7 or 8** further comprising measuring a pulse.

10. The method for measuring blood pressure of any one of claims **7 to 9** comprising providing an acquired blood pressure measurement to a third party device that is different than the second device.

11. The method for measuring blood pressure of claim **10** wherein the step of providing the acquired blood pressure measurement to a third party occurs automatically.

12. The method for measuring blood pressure of claim **10 or 11** wherein the step of providing the acquired blood pressure measurement to a third party device occurs wirelessly.
